# EUROPEAN PATENT APPLICATION

(11) **EP 2 305 114 A1**
(43) Date of publication of application: **06.04.2011**
(21) Application number: 10380101.5
(22) Date of filing: 05.08.2010
(51) Int. Cl.: A61B 5/06, A61B 19/00, G01V 3/08

(54) **Medical system for localising lesions comprising a metallic marker and a metal detector**

(30) Priority: 07.08.2009 ES 200901780
(71) Applicant: Diego Alejandro Utor, Fernández, 11100 San Fernando Cadiz (ES)
(72) Inventor: Diego Alejandro Utor, Fernández, 11100 San Fernando Cadiz (ES)

(57) **Abstract**

In the medical practice in occasions it is necessary to locate and to access a certain anatomical region that must be extirpated surgically or be manipulated. The present invention relates to the combination of a metallic marker having high magnetic permeability and being of small size, which can easily be introduced in the human body using percutaneous puncture or endoscopy, and a metal detector specially adapted to be used in a sterile way.

## Description

This invention consists of a system of medical specialized use of location of secret injuries inside the human body to help surgical extirpation or manipulation, by a previous signposting with a metallic scoreboard of known characteristics in order that it could be found later during the procedure by a metal detector specialized and adapted to that scoreboard, indicated in the general concept of the first recovery.

There are many occasions in the medical practice that it is necessary be able to locate and to accede precisely inside the human body to an anatomical region because an injury has been detected and it must be extirpated surgically or need any type of local manipulation. If the injury is sufficiently big and the region where it places is the easily accessible, this work is not complicated at present.

But every time with more frequency, due to advanced diagnostic technologies, there are detected injuries of minor size placed in recondite places of the human organism. In addition, in occasions, the treatments or manipulations applied to the injury after detection, can make them later undetectable for change size or original characteristics.

In these situations, to locate preciously the mentioned point of the organism to manipulate or to extirpate surgically the injury can be very complex.

There are a lot of methods that the medicine has developed to solve this problem in agreement with the characteristics of the anatomical region to exploring and of the nature of the injury to treating, but still they do not be completely exact, are expensive or are not exempt from risks for the health.

We need a simple and sure method to mark with permanent form the injuries of small size detected inside the human body, in order that they could be located later during the surgical act easily and exactly, avoiding wide or unnecessary resections.

On the other hand, the metal detectors have evolved widely in the last decades and their use has spread to all the areas: the food processing industry and pharmacist, the archaeology, the construction, the warlike industry, safety, etc.

They are based on the detection of small magnetic fields induced in the metallic objects when these enter inside the magnetic field produced by the detector.

There have been designed multiple instruments of different forms and sizes to adapt them to the different utilities, for playful or professional use.

A very used form uses two different bobbins, a transmitter one in which the electricity believes a magnetic field, and a receiving one isolated of the first one, which amplifies the frequencies caught from the magnetic fields provoked in detected objects.

This bobbin amplifies the frequency and sends it to the box of control of the metal detector, where the sensors analyze the sign and generate an acoustic or optical notice

Another possibility is based on the induction of very short and powerful electrical pulses (P.I., pulsate induction) for an alone bobbin that produces a brief magnetic field.

After stopping of sudden form, the magnetic field reverts its polarity turning out to be an ephemeral time of electrical current that circulates along the bobbin (reflected pulsate), just before the emission of the following pulse.

The brief magnetic fields provoked in detected objects, is added to the reflected pulse and that increases its value; this difference with the awaited value is what allows the detection of the metallic secret object. The sign is amplified and transformed into an acoustic or visual notice.

The current technology, according to the condition of the technology, allows to the metal detectors to determine the approximate form the position and the distance which the metallic object is being based on the force of the magnetic generated field. The more nearby the object is, the more loudly it will be the magnetic field that comes proceeding from the object
- type of detected metal and its magnetic permeability, which is not any more than the capacity to attract and to make it pass across it the magnetic fields. This way the materials can qualify between iron-magnetics, that are those of major capacity (iron, cobalt, nickel, ), diamagnetic (gold, silver, bronze, copper, ...) or paramagnéticos (aluminium, wolframio, palladium, ...).
- size of the object; an object of 10 centimeters can produce a sign approximately 60 times louder that a similar object of 2,5 centimeters to the same depth.
- of the material that intervenes between the object and the detector and its magnetic behavior, for example, an object buried in a neutral area can be detected at more distance that if the area is composed of minerals ferromagnetics as the lodestone, pirrotina, ... or mineral conductive wet salts
- form and position of the object; an object with form of plate or ring parallel to the bobbin is detected better than another with a long perpendicular form to this one.

In contraposition to the state of the technology, if we knew in advance the metal that composes the secret and, object therefore, its magnetic behavior, and its form, size and the general characteristics of the material that surrounds it, and we were calibrating the detector of agreement to this information, it would increase considerably the precision of the detector, making its location more exact. It would be even possible to determine better the distance of the object to the detector to facilitate the task of its recovery.

The present invention consists of the profitable development of two different elements that must be use in a complementary form for carrying out its function: on the one hand the development of a metallic scoreboard to indicate and to distinguish a secret injury inside the human body, and for other one of the profitable development of a metal detector designed to locate the specifically above mentioned metallic scoreboard of known characteristics and to lead the manipulation or extirpation of the injury that indicates.

Therefore, in agreement to the invention, first we conceive a metallic piece of intense and known magnetic permeability, of small size, which could be easily introduced in the human body and placed in the interior or in the immediate vicinity of the injury across percutánea puncture guided by methods of diagnosis for image or across natural orifices of the organism wiht endoscopia, and that remains in its place of invariable form and without displacements. And secondly we develop a specially perfected metal detector to detect the metallic piece commented previously, to be used by the sanitary professionals in a sterile way, to be introduced in isolated places of the human organism, in order to be sufficiently exact to guide the manipulation or extirpation the mentioned injuries, and to facilitate this task with acoustic and optical signs.

We are going to describe later each of two complementary inventions, first the scoreboard and then the metal detector.

As a completely original characteristic, different from the known until to the moment by the technology, the scoreboard will be composed for a metal or alloy of great magnetic permeability (preferably ferromagnetic) that makes it easily detectable, and undegradable inside the human body.

In addition, and it seems to be logical, the scoreboard will not have a great size to be able to be introduced easily inside the human body, but simultaneously it will occupy the biggest possible area to facilitate its detection, That is the reason that the more suitable form will be a metallic thin thread that, due folded, allows the possibility of being mounted in needles or other thin instruments to place it in the injury across puncture percutánea or by endoscopia.

In addition the metallic scoreboard must have some way of subjecting itself to the tissue that was placed in, in order to prevent it from moving of its original situation. Since the mentioned metallic piece is going to be placed inside the organism, it is an indispensable condition that suffers a process of sterilization for power used with asepsis in a surgical procedure. The latter characteristics are similar to other components developed with medical purposes created with another aim different to that of its electromagnetic location.

But the unique characteristics of the alloy of the scoreboard, together with its predetermined size and form, all known, are developments of the state of the technology skill that get advantages to calibrate the detector and to maximize its precision, but we will approach this question when we described the detector.

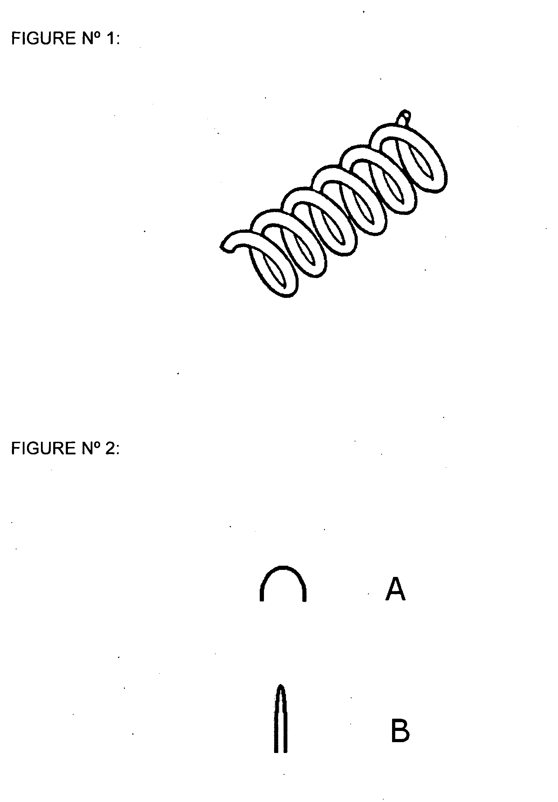

With all these characteristics, and as example of accomplishment, in case the injury was inside a massive or parenchymal organ , the scoreboard will be a wire coiled in form of wharf with a length near to its diameter (FIGURE 1) of about a centimeter. And as example of accomplishment, in case the injury was in a hollow viscera or on the mucous, the scoreboard of metallic ferromagnética and stainless alloy will have the form of a clamp, which two sharp ends that can be closed to pressure on it, catching the tissue on which they place, to hold itself on permanent form (FIGURES- 2 Draw A with the opened clamp and Draw B with the closed clamp). It will measure about a centimeter its longer dimension.

As it has been commented, the metal detector is a specially profitable development of this instrument in order to locate as exact as possible, the previously described metallic scoreboard and to drive and facilitate the surgical extirpation or medical manipulation of the secret injured before marked . First we adapt the instrument to the surgically way that is going to be in use, add a series of technical specific improvements for this use and we incorporate all the possible improvements in the design in order to optimize the comfort of the operator and the global performance of the system. All these perfected characteristics are not present in the condition of the technology, they will be explain later when the invention is described.

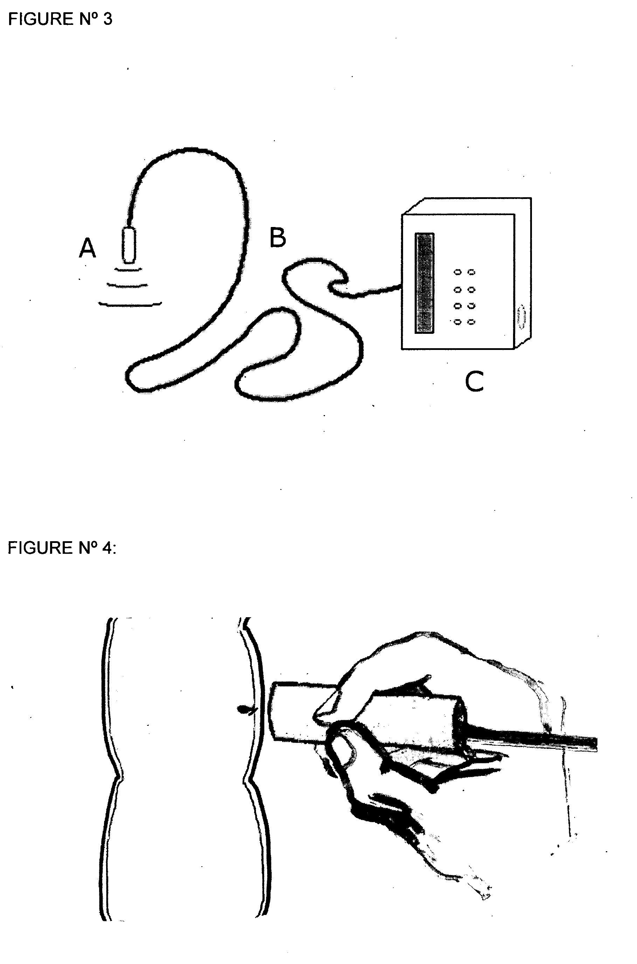

The metal detector would be composed of three principal parts: Probe, Cable and Base (FIGURE N° 3). The probe that contains the bobbin that emits the sign, the cable that transmits the above mentioned sign and the base where it is amplified, is interpreted and transforms in image and sound, beside containing the system of electrical supply.

The probe (Draw A of the FIGURE 3) will be portable, of small size, which allows it to be handled easily by one hand. It will have preferably a cylindrical, thin form, of approximately 2 to 4 centimeters of diameter and approximately 12 to 15 centimeters length, so that it fits well in one hand and could be handled in the same way as a thick felt-tip pen. This would allow to get to narrow and isolated places from the interior of the human body during a surgical intervention if necessary. It will be composed by a hollow framework realized from hard plastic, resistant to blows or falls, with the minor possible quantity of pieces linked each other watertight, to avoid the entry of blood or other corporal fluids, or water if it was necessary to wash it.

In one of its ends it will be connected with the following component, the cable, in form equally suspends and with an electrical suitable isolation. At the other end the probe will be closed. This end is the most important of the device since it has the bobbin. The design of this piece will' be adapted to the framework that contains it and to the technical characteristics proposed to optimize its performance.

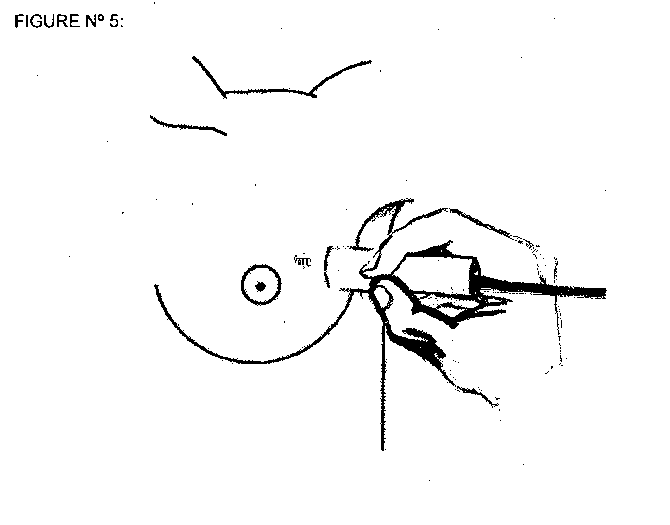

In agreement with all the described characteristics, according to an example of accomplishment, its managing would be as will be described in the FIGURE N° 4 used to locate a clamp in a hollow viscera as the colon, and in the FIGURE N° 5 used to locate a wharf inside a massive organ as the breast, so that it serves to aim at the sensitive end of the probe with a hand to the search of the metallic scoreboard.

In a specially profitable development, the probe can be designed by a major length (approximately 30 centimeters) and a minor diameter (of approximately 1 centimeter) to adapt it to the specific use of the laparoscópic surgery and its ports of access.

The cable (Drawing B of the FIGURE 3) will be sufficiently long in order that it could join the wide form of the probe that will place inside the surgical sterile field, and the base that must be placed distant view the above mentioned field, having at least approximately 300 centimeters long. It must be also semiflexible to allow its mobility across the interior of the organism. The connections with both devices to every end of the cable it will be suspended and it will have an electrical suitable isolation.

Both the probe and part of the cable are going to enter in touch in an sterile way. In order to do not to have to sterilize the equipment in every use, there exist on the market a few sterile cases of transparent plastic of one use of approximately 250 centimeters long that will be adapted perfectly to these components, covering them and isolating them of the surgical way without interfering in its function.

The base (Drawing C of the FIGURE 3), where it will connected the another end of the cable will be equally portable in order that it could be moved easily. To facilitate this task it can benefit using rechargeable batteries, in order that it is not indispensable that he has to be connected to the electrical net all the time It will be composed by a hollow framework constructed of hard plastic in the shape of a resistant to blows bucket and broad support to prevent its fall in case of extract inadvertently the cable. We believe the most adapted type of metal detector for the characteristics of our invention is an induction of pulses one (P.I., touch induction) than an alone pay-out reel uses and receptora, but it is possible to adapt other types of technology to carry out the same performance.

Inside the framework of the base all the components of the detector will be contained, except the inducting bobbin of the pulses that is in the probe. They will not be different from those who exist on the market, and according to the best existing advances it is possible to determine the approximate form of he distance that exists from the detector to a metallic unknown object, warning with sonorous and visual signs to facilitate the operation. Our invention profitably has the aptitude to adapt its estimations (fitting adequately the frequency and the intensity of the issued emitted sign and the susceptibility of the sensors that process the sign to the known properties about the scoreboard before described (the magnetic characteristics of the metal of the scoreboard and its form and size), as well as to the way in which we are going to look (major or minor density of the organ, its thickness or proportion of blood and other physiological liquids) by a previous process meticulous calibrated, that we can increase the accuracy of the location with

In this calibration of the detector, or in another form, in the coupling among the object to detecting, the way and the detector, there is based great part of the advantages of this development. For it, the base has in one of its faces a panel of controls where it can be able to modify these variables mentioned in the detector. In addition, the same face will have several optical indicators or a screen where there will appear the selected variables together with indicators that facilitate the interpretation of the sign got for the operator of the instrument. To this will be added the acoustic signs to the sign received of the scoreboard in order that it is not indispensable to turn the look of the operative field to interpret the results.

According to an example of accomplishment, the base will have the form that can estimate in the Drawing C of the FIGURE N ° 3; in its frontal part it will have the control panel with the controls, the optical indicator and the screen, and in its lateral part the loudspeakers for the acoustic notices. The controls will include the button of ignition, the possibility of modifying the variables of search according to the type of scoreboard (to choosing between the type clamps or soft type, and to being able to choose different types of ferromagnetic alloy according to one be more detectable in a few organs that in others), to modify-the variables of search according to the type of fabric in that is the scoreboard (a hollow viscera of slightly thick walls, as stomach or colon, or a massive and more thickness organ, and inside the latter it might continue cataloguing as it being a question of an organ of dense fabric, since it can be the breast, or lighter one as the lung

Also between the controls it will be possible to change the volume of the acoustic sign and choose between different alarms, in order that the operator could choose the most perceptible for him in an environment of noises as, the operating room. To help to extract conclusions on the proximity of the scoreboard, the sound will be able to change of tone and intensity according to the quiet sign, being a tenuous and serious sound if the sign is scanty and the distance to the big scoreboard, and more intense and acute the more nearby one finds the scoreboard.

The optical indicator will complement the characteristics described by the acoustic sign with a vertical line of lights of size and proportional colour to the value of the measurement. The lights of the low part of the bar will be small and green, those of the half will be yellow and the superiors, of major size and of red colour, they will be igniting of progressive form, from the bottom up the more intense that is detected and more nearby the scoreboard is

On the screen there will appear the levels of load of the batteries and all the adjustments selected for the search. To change them there will be able to be opened several menus with which to select the specifications most according to the search that is going to be realized according to the variables commented previously.

On the same screen, it will appear with characters of great size and in an outstanding place, the distance in centimeters from the end of the probe to the scoreboard. Under these numbers that will change during the detection with the movements of the probe, of profitable form, there will appear the minor distance detected and memorized by the system. This utility can serve to value the margins of healthy fabric with which there is extirpated the injury, which so important they are in the malignant extirpation of tumors. They will be able to show on the screen all kinds of usefulness to optimize the performance of the system.

Certainly, this design will be able to adjust in conformity with the commercial regulations of the countries where it is commercialized, to the international procedure for the Electrical Security and the Electromagnetic Compatibility (EMC), as well as to the safety procedure of the electromedical devices that apply in this place.

Described sufficiently the nature of the invention, as well as the way of realizing it in the practice, must certify that the dispositions previously indicated are capable of modifications of detail in all that they do not alter its fundamental beginning.

Though in the text we refer almost always to medical applications to use in the human being, it is evident that this use can spread in the same conditions for its application in any alive being (in the veterinary practice, in investigation, etc.), and its fundamental principles will not be altered

## Claims

1. Medical specialized system of use for the location of secret injuries inside the human body in order to help its surgical removal or its manipulation, **characterized by** the combined and complementary use of a metallic Scoreboard that is placed on the above mentioned injury, and a metal detector that allows the location of the above mentioned Scoreboard, and consequently, the injury.

2. Medical specialized system of use for the location of secret injuries inside the human body to help to its surgical removal or its manipulation according to the recovery 1, characterized for the metallic Scoreboard characteristics, as the composition, the size, the form, have been developed specifically to facilitate its detection with the metal detector inside alive tissues, and, the form and the technical adjustments of the metal detector have been developed to be used specifically with the above mentioned metallic Scoreboard of known characteristics, hereby, to increase the precision of the location.

3. Medical specialized system of use for location of secret injuries inside the human body to help to its surgical removal or its manipulation according to the recovery 1 and 2, characterized because the metallic Scoreboard is composed by a metal ferromagnetic alloy that awards an intense magnetic permeability, makes it stainless and undegradable inside the human body and allows it to support the habitual methods of sterilization used with asepsis.

4. Medical specialized system of use for location of secret injuries inside the human body to help to its surgical removal or its manipulation according to the recovery 3 , characterized because the metallic Scoreboard has the form of a metallic thin wire of small size, coiled or folded of sharp ends, in order that, it could be introduced easily in the human body inside the injury that is tried to be marked using systems of percutaneus puncture represented by methods of diagnosis by image or any endoscopic method, once placed, it could be fixed to the tissues to avoid its mobilization.

5. Medical specialized system of use for location of secret injuries inside the human body to help to its surgical removal or its manipulation according to the recovery 1 and 2 , characterized because the metal detector is portable and adapted to its use in a surgical way and used in narrow and difficult places of access of the human body, and tha is the reason that the habitual components that form a metal detector are distributed between three different pieces that compose these detector and connected: the Probe, the Cable and the Base (FIGURE N ° 3).

6. Medical specialized system of use for location of secret injuries inside the human body to help to its surgical removal or its manipulation according to the recovery 5th, characterized because the Probe of the metal detector is composed by a hollow and suspended framework of hard and resistant plastic with elongated portable cylinder form, that fits and manages easily with one hand, that can get in narrow and isolated places in the interior of the human body during a medical or surgical procedure.

7. Medical specialized system of use for location of secret injuries inside the human body to help to its surgical removal or its manipulation according to the recovery 5th and 6th, characterized because the Probe of the metal detector in his its inside presents, in one of its ends, the bobbin of the metal detector, and in the opposite end it connects with the other component of the detector, the Cable.

8. Medical specialized system of use for location of secret injuries inside the human body to help to its surgical removal or its manipulation according to the recovery 5th to 7th, characterized because the Cable of the metal detector is sufficiently long to join widely the Probe that will be in use inside the surgical sterile field and the Base that must be placed in the mentioned field, that is, at least approximately 300 cm.

9. Medical specialized system of use for location of secret injuries inside the human body to help to its surgical removal or its manipulation according to the recovery 5th to 8th, characterized because the Cable of the metal detector is semi-flexible to allow the maximum resistance and mobility for the surgical field.

10. Medical specialized system of use for location of secret injuries inside the human body to help to its surgical removal or its manipulation according to the recovery 5^{a} to 9^{a}, characterized because the connections with the devices to each of its ends of the Cable of the metal detector, are suspended and they have an electrical isolation adapted to allow its cleanliness in case of be in touch with corporal fluids.

11. Medical specialized system of use for location of secret injuries inside the human body to help to its surgical removal or its manipulation according to the recovery 5th to 10th, characterized because the Base of the metal detector, which is joined to the Cable, is portable and composed by a hollow framework of hard plastic inside which the habitual components of a metal detector, exempting the bobbin, which places in the Probe and that connects with the Cable.

12. Medical specialized system of use for location of secret injuries inside the human body to help to its surgical removal or its manipulation according to the recovery 5th to 11th, characterized because the metal detector has the aptitude to fit and calibrate the frequency and the intensity of the emitted sign and the susceptibility of the sensors that process the caught sign, to the combination that offers the major precision according to the type of Scoreboard used (in case of one or more different types with different magnetic characteristics, form or sizes adapted to different territories of the human body) and to the way we are going to look for (major or minor density of the fabrics of the organ in which the Scoreboard will be placed, its thickness or proportion of blood and other physiological liquids), in order to obtain the major performance of the detector in every concrete situation.

13. Medical specialized system of use for location of secret injuries inside the human body to help to its surgical removal or its manipulation according to the recovery 5th to 12th, characterized because the metal detector emits diverse acoustic and visual signs that will change of intensity in a directly proportional form to the quiet sign, or, in an indirectly proportional form to the distance of the Probe from the metal detector to the Scoreboard, to facilitate the task to the operator of the system, including the aptitude to show in a true form on a screen, the distance in centimeters that separates the Probe of the metallic Scoreboard after to fit the detector according to the type of the used Scoreboard and the tissue where is lodged.

14. Veterinary specialized use system of location for secret injuries inside the body of the animals to help to its surgical removal or its manipulation according to the previous recoveries.
